# EUROPÄISCHE PATENTSCHRIFT

(11) **EP 1 749 572 B1**
(45) Veröffentlichungstag und Bekanntmachung des Hinweises auf die Patenterteilung: **08.08.2018**
(21) Anmeldenummer: 06114906.8
(22) Anmeldetag: 02.06.2006
(51) Int. Cl.: B01J 23/75, B01J 23/755, B01J 21/04, B01J 35/10, C07C 29/141

(54) **VERFAHREN ZUR HYDRIERUNG VON OXO-ALDEHYDEN MIT HOHEN ESTERGEHALTEN**
PROCESS FOR THE HYDROGENATION OF OXO-ALDEHYDES WITH A HIGH ESTER CONTENT
PROCÉDÉ D'HYDROGÉNATION D'OXO-ALDÉHYDES À HAUTE TENEUR EN ESTER

(30) Priorität: 30.07.2005 DE 102005035816
(43) Veröffentlichungstag der Anmeldung: 07.02.2007
(73) Patentinhaber: Evonik Degussa GmbH, 45128 Essen (DE)
(72) Erfinder: Kaizik, Alfred, 45772, Marl (DE); Lüken, Hans-Gerd, 45770, Marl (DE); Graß, Michael, 45721, Haltern am See (DE); Maschmeyer, Dietrich, 45657, Recklinghausen (DE); Büschken, Wilfried, 45721, Haltern am See (DE)

(56) Entgegenhaltungen:
- EP-A2- 1 219 584
- DE-A1- 4 310 971
- DE-A1- 19 933 348
- GB-A- 2 142 010
- US-A- 4 950 633

## Beschreibung

Die vorliegende Erfindung betrifft ein Verfahren zur Herstellung von Alkoholen durch Hydrierung von Hydroformylierungsgemischen mit hohen Estergehalten in flüssiger Phase und Katalysatoren dafür.

Alkohole können durch katalytische Hydrierung von Aldehyden, die beispielsweise durch Hydroformylierung von Olefinen erhalten worden sind, hergestellt werden. Große Mengen von z. B. auf diese Weise hergestellten Alkoholen werden als Lösungsmittel und als Zwischenprodukte für die Herstellung vieler organischer Verbindungen verwendet. Wichtige Folgeprodukte von Alkoholen sind Weichmacher und Detergenzien.

Es ist bekannt, Aldehyde katalytisch mit Wasserstoff zu Alkoholen zu reduzieren. Dabei werden häufig Katalysatoren eingesetzt, die mindestens ein Metall aus den Gruppen Ib, IIb, VIa, VIIa, und/oder VIIIa des Periodensystems der Elemente enthalten. Die Hydrierung von Aldehyden kann kontinuierlich oder diskontinuierlich mit pulverförmigen oder stückigen Katalysatoren in der Gas- oder Flüssigphase durchgeführt werden.

Für die technische Herstellung von Alkoholen durch Hydrierung von Aldehyden aus dem Oxo-Prozess (Hydroformylierung von Olefinen) werden, vor allem bei Großprodukten, kontinuierliche Verfahren mit im Festbett angeordneten Katalysatoren in der Gas- oder Flüssigphase bevorzugt.

Im Vergleich zur Gasphasenhydrierung weist die Flüssigphasenhydrierung die günstigere Energiebilanz und die höhere Raum-Zeit-Ausbeute auf. Mit steigender Molmasse des zu hydrierenden Aldehyds, d. h. mit steigenden Siedepunkten, nimmt der Vorteil der günstigeren Energiebilanz zu. Höhere Aldehyde mit mehr als 7 Kohlenstoffatomen werden demnach vorzugsweise in der Flüssigphase hydriert.

Die Hydrierung in der Flüssigphase hat allerdings den Nachteil, dass aufgrund der hohen Konzentrationen sowohl an Aldehyden als auch an Alkoholen die Bildung von Hochsiedern durch Folge- und Nebenreaktionen begünstigt wird. So können Aldehyde leichter Aldolreaktionen (Addition und/oder Kondensation) eingehen und mit Alkoholen Halb- oder Vollacetale bilden. Die entstandenen Acetale können unter Abspaltung von Wasser oder Alkohol Enolether bilden, die unter den Reaktionsbedingungen zu den gesättigten Ethern hydriert werden. Durch die Bildung dieser sekundären Nebenprodukte wird die Ausbeute vermindert. Die als Hochsieder bezeichneten Nebenprodukte können bestenfalls teilweise in nachgeschalteten Anlagen in Wertprodukte, wie Ausgangsaldehyde und Zielalkohole, zurückgespalten werden.

Technische Aldehydgemische, die zur Hydrierung eingesetzt werden, enthalten häufig bereits Hochsieder in unterschiedlicher Konzentration.

Bei der Hydroformylierung von Olefinen in Gegenwart von Kobalt- und RhodiumKatalysatoren werden Rohaldehyde erhalten, die neben Aldolprodukten, Ethern und Acetalen auch Ester, insbesondere Ameisensäureester (Formiate), als Hochsieder enthalten. Werden diese Gemische in der Gasphase hydriert, so kann der größte Teil der Hochsieder im Verdampfer abgetrennt und in einem separaten Verfahrensschritt zu Wertprodukten aufgearbeitet werden.

Bei der Flüssigphasenhydrierung dagegen bleiben die Hochsieder im Reaktorzulauf. Sie werden in der Hydrierstufe zum größten Teil zu Produkten hydriert, aus denen kein Wertprodukt mehr gewonnen werden kann. Im Reaktorzulauf enthaltene Ester werden häufig nicht zu den entsprechenden Zielalkoholen umgesetzt.

In US 5,059,710 wird die Ausbeute an Alkoholen durch Hydrierung von Rohaldehyden dadurch erhöht, dass in einer der Hydrierung vorgeschalteten Verfahrenstufe ein Teil der Hochsieder bei erhöhter Temperatur mit Wasser zu Aldehyden oder Alkoholen zurückgespalten wird. Hydrolyse und Hydrierung sind daher getrennte Verfahrensstufen, wobei dem Dokument keine Angaben über den Wassergehalt des zu hydrierenden Gemischs zu entnehmen sind.

Ein ähnliches Verfahren wird in US 4 401 834 offenbart. Auch hier erfolgt die Spaltung von Hochsiedern in Gegenwart von Wasser vor dem eigentlichen Hydrierungsschritt.

In GB 2 142 010 wird ein Verfahren zur Hydrierung von rohen Aldehyden mit 6 bis 20 C-Atomen, die Hochsieder und geringe Mengen an Schwefelverbindungen enthalten, zu den entsprechenden gesättigten Alkoholen beansprucht. Die Hydrierung erfolgt in zwei hintereinander geschalteten Reaktoren. Der erste Reaktor enthält einen MoS₂/C- und der zweite Reaktor einen Ni/Al₂O₃-Katalysator. Die Hydrierung wird in beiden Reaktoren unter Zusatz von bis zu 10 % Wasserdampf, bezogen auf den Eduktstrom, im Temperaturbereich 180 bis 260 °C und einem Wasserstoffpartialdruck von 15 bis 21 MPa mit großem Wasserstoffüberschuss durchgeführt. Dieser ist nach den Beispielen so groß, dass sich das zugesetzte Wasser praktisch nur in der Gasphase befindet. Das Ziel dieses Verfahrens liegt in der Zurückdrängung der Bildung von Kohlenwasserstoffen durch Hydrogenolyse der Alkohole. Über eine Zunahme bzw. Abnahme von Hochsiedern und Formiaten bei der Hydrierung werden keine Aussagen gemacht.

In US 2 809 220 wird eine Flüssigphasenhydrierung von Hydroformylierungsgemischen in Gegenwart von Wasser beschrieben. Als Katalysator werden schwefelhaltige Katalysatoren eingesetzt. Die Hydrierung wird im Druckbereich von 10,5 bis 31,5 MPa und im Temperaturbereich von 204 bis 315 °C in Gegenwart von 1 bis 10 % Wasser, bezogen auf das Edukt, durchgeführt. Um das zugesetzte Wasser in der Gasphase zu halten, wird ein großer Überschuss an Wasserstoff (892 bis 3566 Nm³ Wasserstoff je m³ Edukt) eingesetzt. Bezüglich des hohen Wasserstoffüberschusses wird auf die Diskussion von GB 2 142 010 verwiesen. Nachteilig an diesem Verfahren ist weiterhin der hohe spezifische Energieverbrauch.

Ein weiteres Verfahren zur Hydrierung von Hydroformylierungsgemischen offenbart DE 198 42 370. Hier wird beschrieben, wie Hydroformylierungsgemische in der Flüssigphase an Kupfer, Nickel und Chrom enthaltenden Trägerkatalysatoren hydriert werden können. Je nach Herstellverfahren der Hydroformylierungsgemische (Rhodium- oder Kobaltverfahren) enthalten diese Gemische Wasser. Das offenbarte Verfahren ist für die selektive Hydrierung der Aldehyde zu Alkoholen, ohne Hydrierung der in der Hydroformylierung nicht umgesetzten Olefine ausgelegt, d. h. die Hochsieder (vor allem Acetale) werden nicht zu Wertprodukt umgesetzt. Dies ist wirtschaftlich ungünstig und daher verbesserungsfähig.

In DE 100 62 448 wird ein Verfahren zur Hydrierung von Aldehyden mit Estergehalten bis 5 Massen-% zu den entsprechenden Alkoholen beschrieben. Die Hydrierung wird in flüssiger Phase in Gegenwart von homogen gelöstem Wasser durchgeführt. Als Katalysatoren werden vorzugsweise kupferhaltige Trägerkatalysatoren eingesetzt. Bei diesem Verfahren werden Aldehyde praktisch ohne Bildung von Hochsiedern zu den entsprechenden Alkoholen hydriert. Es besteht jedoch der Nachteil, dass die Ester nur langsam zu den entsprechenden Alkoholen umgesetzt werden, sodass sich bei hoher Selektivität nur eine geringe Raum-Zeit-Ausbeute ergibt.

In DE 35 42 595 wird für die Hydrierung von esterhaltigen Hydroformylierungsgemischen ein zweistufiges Hydrierverfahren beschrieben. Dabei wird in der ersten Hydrierstufe ein Trägerkatalysator mit Siliziumoxid als Träger sowie Nickel und Molybdän als hydrieraktive Komponenten und in der zweiten Stufe ein Katalysator, der die hydrieraktiven Metalle Kobalt, Kupfer, Mangan und Molybdän enthält, eingesetzt. Der Hauptnachteil dieses Verfahren besteht darin, dass, um gute Ausbeuten zu erhalten, Hydrierdrucke von 25 MPa angewendet werden.

In DE 199 33 348 A1 wird ein Verfahren zur Reduktion von nickelhaltigen Katalysator-Vorläufern für die Aldehydhydrierung in der Flüssigphase beschrieben. Die Reduktion der Katalysator-Vorläufer wird hier in einem einstufigen Prozess vorgenommen. Die geschieht vorzugsweise im gleichen Reaktor wie die nachfolgende Aldehydhydrierung. Die Reduktion erfolgt in der Flüssigphase mit Wasserstoff.

In der EP 1 219 584 A2 wird ein Verfahren zur Hydrierung von Reaktionsgemischen aus der Hydroformylierung von Olefinen mit 4 bis 16 Kohlenstoffatomen in der homogenen Flüssigphase an Festbettkatalysatoren beschrieben. Die Festbettkatalysatoren enthalten mindestens ein Element der achten Nebengruppe. Die Flüssigphase des Reaktorauftrages enthält zwischen 0,05 und 10 Gew.-% Wasser.

Da die bekannten Hydrierverfahren hinsichtlich der Wirtschaftlichkeit (geringer Kapitaleinsatz, hohe Produktausbeute, hohe Raum-Zeit-Ausbeute, geringer Energieaufwand) für die Umsetzung von Aldehydgemischen mit hohen Estergehalten zu den entsprechenden Alkoholen nicht optimal sind, bestand die Aufgabe, ein verbessertes Hydrierverfahren bereitzustellen.

Es wurde nun überraschend gefunden, dass für die Hydrierung von esterhaltigen Aldehyden zu den entsprechenden Alkoholen Trägerkatalysatoren mit γ-Aluminiumoxid als Träger, das eine BET-Oberfläche von 70 bis 350 m²/g aufweist, und als hydrieraktives Metall Nickel oder Kobalt oder eine Kombination davon aufweist, hervorragend geeignet sind. Die erfindungsgemäßen Katalysatoren hydrieren die Einsatzstoffe mit hoher Selektivität und mit hoher Raum-Zeit-Ausbeute zu den entsprechenden Alkoholen. Dies war sehr überraschend, da insbesondere von Nickelkatalysatoren bekannt ist, dass an ihnen Nebenreaktionen ablaufen, beispielsweise Etherbildung oder Überhydrierung der Zielalkohole zu den entsprechenden Alkanen.

Gegenstand der vorliegenden Erfindung ist deshalb ein Verfahren zur katalytischen Hydrierung von esterhaltigen Aldehydgemischen zu den entsprechenden Alkoholen,
dadurch gekennzeichnet,
dass ein Trägerkatalysator, der als Trägermaterial γ-Aluminiumoxid mit einer BET-Oberfläche von 70 bis 350 m2/g und als hydrieraktive Komponente 15 bis 25 Massen-% Nickel und/oder 5 bis 30 Massen-% Kobalt bezogen auf die Gesamtmasse des reduzierten Katalysators aufweist, wobei der Katalysator frei von Cu, Ru, Rh, Pd, Ag, Au, Pt, Ir und/oder Os ist und dass als esterhaltiges Aldehydgemisch ein Reaktionsgemisch aus der Hydroformylierung von Olefinen mit 6 bis 20 C-Atomen, welches einen Estergehalt von 3 bis 15 Massen-% aufweist, eingesetzt wird.

Das erfindungsgemäße Verfahren zur Hydrierung von Ester-haltigen Aldehydgemischen, die insbesondere bei der Hydroformylierung von Olefinen entstehen, zu den entsprechenden Alkoholen, bei dem der erfindungsgemäße Katalysator eingesetzt wird, weist eine Reihe von Vorteilen auf. So ist die Selektivität der Alkoholbildung im erfindungsgemäßen Verfahren hoch. Zudem entstehen in dem erfindungsgemäßen Verfahren kaum Hochsieder, beispielsweise durch Acetalbildung, Aldoladdition, Aldolkondensation und Folgereaktionen. In dem erfindungsgemäßen Verfahren werden sowohl die Aldehyde als auch die Ester in hohen Raum-Zeit-Ausbeuten zu den entsprechenden Alkoholen umgesetzt. Im Unterschied zu bekannten Verfahren kann das erfindungsgemäße Verfahren bei relativ geringen Drücken durchgeführt werden.

Nachfolgend wird die Erfindung beispielhaft beschrieben, ohne dass die Erfindung, deren Schutzbereich sich aus den Ansprüchen und der gesamten Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche zum Offenbarungsgehalt der vorliegenden Erfindung gehören, ohne dass diese aus Gründen der besseren Übersichtlichkeit explizit genannt worden sind.

Der erfindungsgemäße Hydrierkatalysator, der vorzugsweise zur Hydrierung von esterhaltigen Aldehydgemischen verwendet wird, zeichnet sich dadurch aus, dass er als Trägermaterial ein γ-Aluminiumoxid mit einer BET-Oberfläche von 70 bis 350 m²/g, vorzugsweise von 110 bis 250 m²/g (bestimmt nach dem BET-Verfahren durch Stickstoff-Adsorption gemäß DIN ISO 9277) und als hydrieraktive Komponente Nickel und/oder Kobalt aufweist. Das mittlere Porenvolumen (bestimmt durch Quecksilber-Porosimetrie nach DIN 66133) beträgt vorzugsweise von 0,5 und 0,8 cm³/g.

Die im Folgenden angegebenen Zusammensetzungen beziehen sich auf die reduzierten Katalysatoren. Der erfindungsgemäße Katalysator enthält bevorzugt von 15 bis 25 Massen-% Nickel und/oder von 15 bis 25 Massen-% Kobalt. Bevorzugt enthält der erfindungsgemäße Katalysator von 17,5 bis 22,5 Massen-% Kobalt. Zwar werden auch mit Katalysatoren, die von 17,5 bis 22,5 Massen-% Nickel aufweisen, die Ester ähnlich gut wie mit dem Kobaltkatalysator abgebaut, bei sinkender Konzentration an Estern kann es aber zu Ausbeuteverlusten an Alkohol kommen, wenn die Hydrierung lange bei niedrigen Esterkonzentrationen durchgeführt wird. Vorzugsweise sind die erfindungsgemäßen Katalysatoren frei von Kupfer oder Edelmetallen, insbesondere frei von Cu, Ru, Rh, Pd, Ag, Au, Pt, Ir und/oder Os.

Optional können die erfindungsgemäßen Katalysatoren Alkalimetall-, Erdalkalimetall- oder Zinkverbindungen enthalten. Der Anteil dieser Verbindungen (als Metalloxid berechnet) beträgt vorzugsweise von 0,01 bis 3 Massen-%, bevorzugt von 0,05 bis 1 Massen-%. Weiterhin können die Katalysatoren optional Verarbeitungshilfsmittel, wie z. B. Graphit enthalten.

Die erfindungsgemäßen Katalysatoren können durch Auftragen von mindestens einem Metall ausgewählt aus Kobalt oder Nickel auf ein geeignetes γ-Aluminiumoxid hergestellt werden. Geeignetes Trägermaterial ist z. B. unter den Handelsnamen SP E538 der Fa. Axens und Alcoa LD 350 der Fa. Alcoa erhältlich. Als geeignete Trägermaterialien werden vorzugsweise solche eingesetzt, die ein mittleres Porenvolumen von 0,5 bis 0,8 cm³/g aufweisen. Das Aufbringen des zumindest einen Metalls kann durch Behandeln des Trägers mit geeigneten Metallverbindungen selbst, mit einer Lösung oder Dispersion von Metallverbindungen oder mit Metallsalzlösungen, insbesondere wässrigen Metallsalzlösungen, der entsprechenden Metalle erfolgen. Die Behandlung kann z. B. durch ein Tränken des Trägers in oder mit einer der genannten Lösungen oder Dispersionen oder durch ein Besprühen des Trägers mit einer der genannten Lösungen oder Dispersionen oder durch andere geeignete Verfahren erfolgen. Als Metallverbindungen eignen sich beispielsweise Nitrate, Halogenide, Carbonate, Carboxylate, Acetylacetonate der Metalle und deren lösliche Metallkomplexe, beispielsweise Amminkomplexe. Als gegebenenfalls vorhandenes Lösemittel können beispielsweise Alkohole oder Wasser verwendet werden. Als bevorzugtes Lösemittel wird Wasser eingesetzt. Sollen Katalysatoren hergestellt werden, bei denen mehr als ein Metall aufgetragen wird, können die entsprechenden Metallverbindungen gleichzeitig oder nacheinander aufgebracht werden.

Die Menge der eingesetzten Metallverbindungen bzw. die Menge und Konzentration der eingesetzten Lösungen oder Dispersionen von Metallverbindungen, insbesondere von Metallsalzen werden jeweils so bemessen, dass der gewünschte Massenanteil der hydrieraktiven Komponente im Katalysator erreicht wird.

Die mit einer Metallverbindung oder einer Lösung oder Dispersion einer Metallverbindung, insbesondere einer Metallsalzlösung behandelten, also insbesondere beschichteten bzw. getränkten Träger werden anschließend vorzugsweise bei einer Temperatur von 200 bis 600 °C calziniert. Es kann vorteilhaft sein, wenn vor dem Calzinieren der behandelte Träger zunächst getrocknet wird. Das Trocknen erfolgt vorzugsweise bei einer Temperatur von 80 bis 150 °C. Erfolgt die Behandlung des Träger in mehreren Schritten, z. B. durch mehrmaliges Tränken oder Besprühen, so kann es vorteilhaft sein, wenn nach jedem Behandlungsschritt ein wie oben beschriebener Trocknungsschritt und/oder Calzinierschritt durchgeführt wird. Wird nicht nur eine hydrieraktive Komponente auf den Träger aufgebracht, ist die Reihenfolge, in der die hydrieraktiven Komponenten aufgebracht werden, frei wählbar.

Optional kann die Aufbringung der hydrieraktiven Komponenten, Trocknung und Calzinierung in einem Arbeitsgang erfolgen, beispielsweise durch Aufsprühen einer wässrigen Metallsalzlösung auf den Träger bei Temperaturen über 200 °C.

Die erfindungsgemäßen Katalysatoren werden vorzugsweise in eine Form gebracht, die bei der anschließenden Verwendung, z. B. bei der Hydrierung, einen geringen Strömungswiderstand bietet, wie beispielsweise Tabletten, Zylinder, Strangextrudate oder Ringe. Die Formgebung kann dabei wahlweise an verschiedenen Stellen der Katalysatorherstellung, insbesondere vor oder nach dem Calzinieren erfolgen.

Das erfindungsgemäße Verfahren zur katalytischen Hydrierung von esterhaltigen Aldehydgemischen zu den entsprechenden Alkoholen, zeichnet sich dadurch aus, dass ein Trägerkatalysator, der als Trägermaterial γ-Aluminiumoxid mit einer BET-Oberfläche von 70 bis 350 m²/g, vorzugsweise 110 bis 250 m²/g und als hydrieraktive Komponente Nickel und/oder Kobalt aufweist, eingesetzt wird.

Besonders bevorzugt wird der wie oben beschriebene erfindungsgemäße Katalysator in dem erfindungsgemäßen Verfahren eingesetzt. Der eingesetzte Katalysator enthält, bezogen auf die Gesamtmasse des reduzierten Katalysators, besonders bevorzugt von 15 bis 25 Massen-% Nickel und/oder von 15 bis 25 Massen-% Kobalt. Neben diesen Komponenten kann der erfindungsgemäß eingesetzte weitere Komponenten, wie oben angegeben enthalten. Das Trägermaterial weist vorzugsweise ein mittleres Porenvolumen von 0,5 bis 0,8 cm³/g auf.

Im erfindungsgemäßen Verfahren kann die Hydrierung an suspendierten oder stückigen im Festbett angeordneten Katalysatoren kontinuierlich oder diskontinuierlich durchgeführt werden. Bevorzugt wird im erfindungsgemäßen Verfahren eine kontinuierliche Hydrierung an einem im Festbett angeordnetem Katalysator, bei dem sich unter Reaktionsbedingungen die Produkt/Eduktphase hauptsächlich im flüssigen Zustand befindet, durchgeführt.

Wenn die Hydrierung kontinuierlich an einem im Festbett angeordneten Katalysator durchgeführt wird, ist es zweckmäßig, den Katalysator vor der Hydrierung in die aktive Form zu überführen. Dies kann durch Reduktion des Katalysators mit wasserstoffhaltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, vorzugsweise Rohrreaktoren, adiabatisch oder praktisch isotherm sowie einen oder mehrere adiabatisch und die anderen praktisch isotherm betreiben. Weiterhin ist es möglich, die Hydrierung in einem, in mehreren oder in allen der eingesetzten Reaktoren im geraden Durchgang oder unter Produktrückführung durchzuführen.

Das erfindungsgemäße Verfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, bevorzugt von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann ohne oder vorzugsweise mit einem Lösungsmittel durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

### Beispielsweise können folgende Stoffe als Lösemittel eingesetzt werden:

Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole. Ein weiteres bevorzugtes Lösemittel ist das Hydrierprodukt selbst.

Durch die Verwendung eines Lösemittels kann die Aldehydkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aldehydgehalt im Reaktorzulauf von 1 bis 35 %, besonders bevorzugt von 5 bis 25 %. Der gewünschte Konzentrationsbereich kann bei Reaktoren, die in Schlaufenfahrweise betrieben werden, durch das Kreislaufverhältnis (Mengenverhältnis von rückgeführten Hydrieraustrag zu Edukt) eingestellt werden.

Die erfindungsgemäße Hydrierung kann ohne Zusatz von Wasser zum esterhaltigen Aldehydgemisch durchgeführt werden. Vorzugsweise wird die erfindungsgemäße Hydrierung aber unter Zusatz von Wasser zum esterhaltigen Aldehydgemisch durchgeführt. Die Durchführung der Hydrierung in Gegenwart von Wasser kann z. B. wie in DE 100 62 448 beschrieben erfolgen. Bevorzugt erfolgt die Hydrierung unter Wasserzusatz so, dass im Hydrieraustrag 0,05 bis 10 Massen-% Wasser im flüssigen Hydrieraustrag gelöst ist.

Das erfindungsgemäße Verfahren wird vorzugsweise bei einem Druck von 0,5 bis 25 MPa, bevorzugt von 1 bis 15 MPa und besonders bevorzugt von 1,5 bis 3 MPa durchgeführt. Vorzugsweise wird das erfindungsgemäße Verfahren bei einer Temperatur von 120 bis 220 °C, bevorzugt von 140 bis 210 °C und besonders bevorzugt von 160 bis 190 °C durchgeführt.

Als Hydriergase können beliebige wasserstoffhaltige Gasgemische, die vorzugsweise keine schädlichen Mengen an Katalysatorgiften, wie beispielsweise Kohlenmonoxid oder Schwefelwasserstoff enthalten, eingesetzt werden. Die Verwendung von Inertgasen ist optional, bevorzugt wird reiner Wasserstoff oder Wasserstoff in einer Reinheit von größer 95 %, insbesondere größer 98 % eingesetzt. Inertgasbestandteile können beispielsweise Stickstoff oder Methan sein.

Es kann vorteilhaft sein, wenn die einzelnen Reaktoren mit Frisch-Wasserstoff beschickt werden. Um den Wasserstoffverbrauch und die mit dem Abgas bedingten Austragsverluste zu minimieren, ist es jedoch zweckmäßig, das Abgas eines Reaktors als Hydriergas eines anderen Reaktors zu verwenden. Beispielsweise ist es bei einem Verfahren, das in zwei hintereinander geschalteten Reaktoren durchgeführt wird, vorteilhaft, Frisch-Wasserstoff in den zweiten Reaktor einzuspeisen und das Abgas des zweiten Reaktors in den ersten Reaktor zu leiten. In diesem Falle strömen Einsatzstoff und Hydriergas in entgegengesetzter Reihenfolge durch die Reaktoren. Es ist bevorzugt, den Wasserstoffüberschuss bezogen auf die stöchiometrisch notwendige Menge (unter Berücksichtigung des angestrebten Umsatzes) unter 30 %, insbesondere unter 10 %, ganz besonders unter 5 % zu halten.

Mit Hilfe des erfindungsgemäßen Verfahrens können gesättigte oder olefinisch-ungesättigte Aldehyde zu den entsprechenden gesättigten Alkoholen hydriert werden. Dabei können Aldehyde bzw. Aldehydgemische, die nach den unterschiedlichsten Verfahren, beispielsweise durch Aldolkondensation, gewonnen wurden, eingesetzt werden. Insbesondere werden jedoch Aldehydgemische eingesetzt, die durch Hydroformylierung von Olefinen erhalten wurden. Diese Hydroformylierungsgemische enthalten neben den Aldehyden auch Ester, insbesondere Formiate. Bei der Hydroformylierung von Olefinen bzw. Olefingemischen mit 8 bis 16 C-Atomen und einem Verzweigungsgrad (mittlere Anzahl der Verzweigungen, wobei n-Olefine einen Verzweigungsgrad von 0 aufweisen) von über 1 können bei Umsätzen von über 80 % die Estergehalte im Hydroformylierungsgemisch Werte bis zu 30 Massen-% annehmen. Als esterhaltiges Aldehydgemisch wird bevorzugt ein Reaktionsgemisch aus der Hydroformylierung von Olefinen mit 6 bis 20 C-Atomen, welches einen Estergehalt von 1 bis 25 Massen-%, besonders bevorzugt von 3 bis 15 Massen-% und ganz besonders bevorzugt von 5 bis 10 Massen-% aufweist, eingesetzt.

Edukte für die Herstellung der esterhaltigen Aldehydgemische durch Hydroformylierung können z. B. Olefine mit 6 bis 20 C-Atomen, bevorzugt 8 bis 20 C-Atomen mit end- oder innenständigen olefinischen Doppelbindungen, wie z. B., 1-, 2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylhexen-1, das bei der Dimerisierung von Butenen anfallende Gemisch der isomeren C₈-Olefine (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Pentadecene, Hexadecene, das bei der Tetramerisierung von Butenen anfallende C₁₆-Olefingemisch (Tetrabuten), das bei der Pentamerisierung von Butenen anfallende C₂₀-Olefingemisch (Pentabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge, sein. Ebenfalls können Olefine oder Olefingemische, die durch Fischer-Tropsch-Synthese erzeugt worden sind sowie Olefine, die durch Oligomerisierung von Ethen erhalten wurden oder Olefine, die über Methathesereaktionen zugänglich sind, eingesetzt werden. Bevorzugte Edukte für die Herstellung der Hydroformylierungsgemische sind C₈-, C₉-, C₁₂-, C₁₅-, C₁₆- oder C₂₀-Olefingemische.

Die Olefine können in üblicher Weise hydroformyliert werden. Die Reaktionsgemische der Hydroformylierung können dann als Edukte für das erfindungsgemäße Hydrierverfahren eingesetzt werden. Die Hydroformylierung kann mit Rhodium- oder Kobaltkatalysatoren mit oder ohne komplexstabilisierende(n) Zusätze(n), wie organischen Phosphinen oder Phosphiten erfolgen. Die Temperaturen und Drücke können, je nach Katalysator oder Olefin, in weiten Grenzen variieren. Eine Beschreibung der Hydroformylierung von Olefinen findet sich z. B. bei J. Falbe, New Syntheses with Carbon Monoxide, Springer-Verlag, Heidelberg-New York, 1980, Seite 99 ff. sowie bei Kirk-Othmer, Encyclopedia of Chemical Technology, Band 17, 4. Auflage, John Wiley & Sons, Seiten 902 bis 919 (1996).

Bevorzugt werden im erfindungsgemäßen Verfahren als Aldehydgemische Reaktionsgemische aus der Hydroformylierung von Olefinen mit 8 bis 18 C-Atomen, insbesondere Hydroformylierungsgemische, hergestellt aus C₈-, C₁₂- und C₁₆-Olefinen oder C₈-, C₁₂- und C₁₆-Olefingemischen, hydriert. Besonders bevorzugt werden in dem erfindungsgemäßen Verfahren als Aldehydgemische Reaktionsgemische aus der Hydroformylierung von Di-n-buten, Tri-n-buten und Tetra-n-buten hydriert.

Die Reaktionsgemische der Hydroformylierung werden vor dem Einsatz in dem erfindungsgemäßen Verfahren vorzugsweise zunächst vom Katalysator befreit. Wenn ein Kobaltkatalysator verwendet worden ist, kann dies durch Druckentlastung, Oxidation der im Hydroformylierungsgemisch verbliebenen Kobaltcarbonylverbindungen in Gegenwart von Wasser oder wässriger Säure und Abtrennung der wässrigen Phase geschehen. Entkobaltungsverfahren sind gut bekannt, siehe z. B. J. Falbe, in "New Syntheses with Carbon Monoxide", Springer Verlag (1980), Berlin, Heidelberg, New York, Seite 158 ff.

Die vom Hydroformylierungskatalysator befreiten Reaktionsgemische können je nach dem für die Hydroformylierung verwendeten Katalysatorsystem von 3 bis 40 Massen-%, vorzugsweise von 5 bis 30 Massen-% Leichtsieder enthalten. Diese Leichsieder können hauptsächlich nicht umgesetzte Olefine, daneben die entsprechenden gesättigten Kohlenwasserstoffe sowie von 0,05 bis 5 Massen-% Wasser, von 30 bis 90 Massen-% Aldehyde, von 5 bis 60 Massen-% Alkohole, von 0,5 bis zu 30 Massen-% Ester, hauptsächlich Formiate dieser Alkohole und von 0,5 bis 15 Massen-% Hochsieder aufweisen.

Es sei jedoch betont, dass das erfindungsgemäße Verfahren auch mit Hydroformylierungsgemischen ausgeführt werden kann, deren Zusammensetzung in dieser und/oder jener Beziehung nicht diesen Angaben entspricht. So können beispielsweise vor der Hydrierung die Kohlenwasserstoffe (Olefine und Paraffine) vom Hydroformylierungsgemisch abgetrennt werden.

Das nach dem erfindungsgemäßen Verfahren erhaltende Hydrierprodukt kann destillativ aufgearbeitet werden. Dies kann bei Normaldruck oder vermindertem Druck erfolgen. Bei hochsiedenden Alkoholen (Alkoholen mit mehr als 9 Kohlenstoffatomen) wird die Destillation bei vermindertem Druck bevorzugt. Optional wird der gewonnene Alkohol in mehrere Fraktionen aufgetrennt.

Die nach dem erfindungsgemäßen Verfahren gewonnenen Alkohole können beispielsweise zur Herstellung von Weichmachern oder Detergenzien genutzt werden.

Die folgenden Beispiele sollen die Erfindung weiter erläutern, ohne Schutzbereich zu beschränken, der sich aus der Beschreibung und den Patentansprüchen ergibt.

### Beispiel 1 (Vergleichsbeispiel):

### C₁₃-Aldehydhydrierung in der Flüssigphase bei 2,5 MPa / Cu-Cr-Ni-Katalysator (H14279, Lieferfirma: Degussa, Düsseldorf)

Ein Liter eines Reaktionsaustrags der Rh-katalysierten Hydroformylierung von Tributen mit 15,42 Massen-% C₁₃-Aldehyd und 9,13 Massen-% Estern wurde in einer Kreislaufapparatur bei 180 °C und 2,5 MPa absolut an 100 g eines Cu/Cr/Ni-Katalysators (6,7 Massen-% Cu, 3 Massen-% Ni und 0,7 Massen-% Cr) auf γ-Al₂O₃-Träger in der Flüssigphase hydriert. Die Abgasmenge betrug 1 Nl/h. Die Edukt- und Produkt-Analysen, die gaschromatographisch erfolgten, sind in Tabelle 1 wiedergegeben.

**Tabelle 1:**

| Versuchszeit (Stunden) | C₁₂-KWST (Massen-%) | C₁₃-al (Massen-%) | Formiat (Massen-%) | C₁₃-ol (Massen-%) | Hochsieder (Massen-%) |
|---|---|---|---|---|---|
| 0 | 8,35 | 15,42 | 9,13 | 66,95 | 0,15 |
| 0,5 | 8,61 | 4,21 | 7,44 | 79,54 | 0,20 |
| 1 | 8,64 | 2,74 | 7,23 | 81,17 | 0,22 |
| 3 | 8,72 | 1,76 | 5,40 | 83,45 | 0,67 |

Wie man aus der Tabelle 1 entnehmen kann, werden die Ester bei der Hydrierung von Isotridecanal nur mit mäßiger Ausbeute zum Wertprodukt Isotridecanol gespalten.

### Beispiel 2 (Vergleichsbeispiel):

### C₁₃-Aldehydhydrierung bei 6 MPa / Cu-Cr-Ni-Katalysator (H14279; Lieferfirma: Degussa, Düsseldorf)

Ein Liter eines Reaktionsaustrags der Rh-katalysierten Hydroformylierung von Tributen mit 15,82 Massen-% C₁₃-Aldehyd und 9,53 Massen-% Estern wurde in einer Kreislaufapparatur bei 180 °C und 6 MPa absolut an 100 g eines Cu/Cr/Ni-Katalysators auf γ-Al₂O₃-Träger (Zusammensetzung siehe Vergleichsbeispiel 1) in der Flüssigphase hydriert. Die Abgasmenge betrug 1 N1/h. Die Edukt- und Produkt-Analysen sind in Tabelle 2 wiedergegeben.

**Tabelle 2:**

| Versuchszeit (Stunden) | C₁₂-KWST (Massen-%) | C₁₃-al (Massen-%) | Formiat (Massen-%) | C₁₃-ol (Massen-%) | Hochsieder (Massen-%) |
|---|---|---|---|---|---|
| 0 | 8,20 | 15,82 | 9,53 | 66,25 | 0,20 |
| 0,5 | 8,93 | 2,87 | 4,37 | 82,90 | 0,93 |
| 1 | 8,75 | 2,45 | 3,04 | 84,36 | 1,40 |
| 3 | 8,85 | 1,65 | 1,16 | 86,69 | 1,65 |

Wie man der Tabelle 2 entnehmen kann, werden Ester (Formiate) durch gezielte Steigerung des Reaktionsdruckes von 2,5 auf 6 MPa deutlich schneller zu Wertprodukten gespalten.

### Beispiel 3 : Herstellung von Nickel -Trägerkatalysatoren (gemäß der Erfindung)

Ein kommerzieller Aluminiumoxidträger (SP E538 der Fa. Axens) in Form von zylindrischen Extrudaten mit einem Durchmesser von etwa 1,2 mm und einer Länge von 4 bis 6 mm, der eine BET-Oberfläche von 270 m²/g (bestimmt nach dem BET-Verfahren durch Stickstoff-Adsorption gemäß DIN ISO 9277) und ein Porenvolumen von 0,7 ml/g (bestimmt durch Quecksilber-Porosimetrie nach DIN 66133) aufweist, wurde mit einer wässrigen Co-Salzlösung getränkt.

Für die BET-Oberflächenbestimmung wurde der Aluminiumoxidträger im Vakuum bei 200 °C bis zu einem Restdruck kleiner als 66 mPa aufgeheizt. Unter Verwendung eines Sorptiongerätes der Fa. Micromeritics ASAP 2400 wurde an der entgasten Probe volumetrisch die Adsorptionsisotherme unter diskontinuierlicher Stickstoffzufuhr aufgenommen. Die Auswertung erfolgte durch Mehrpunktbestimmung. Als Adsorptiv wurde Stickstoff mit einer Reinheit von 99,996 % eingesetzt. Für die Bestimmung des Porenvolumens mittels Hg-Porosimetrie wurde ein Gerät von Typ Pascal 140/440 der Fa. Porotec eingesetzt.

Der Katalysatorträger wurde zweimal mit einer wässrigen Nickel(II)hexammincarbonat-Lösung getränkt, deren Nickelgehalt 18 Massen-% betrug. Das beim ersten Tränkvorgang vom Träger aufgenommene Lösungsvolumen entsprach dabei etwa dem Porenvolumen des verwendeten Trägers. Nach der ersten Imprägnierung wurde der getränkte Träger bei 120 °C 12 h lang in Luft getrocknet und anschließend erneut mit der wässrigen Nickel(II)hexaammincarbonat-Lösung getränkt. Beim zweiten Tränkvorgang nahm das Material nur noch etwa 80 % des Porenvolumens des Trägers auf. Nach zwölfstündiger Trocknung bei 120 °C in Luft wurde der Katalysator bei 450 °C 16 h lang in einem Stickstoff/Wasserstoff-Gemisch, dessen Wasserstoffgehalt dabei stufenweise von 5 bis auf 50 Volumen-% erhöht wurde, aktiviert (reduziert). Nach Abkühlen auf Raumtemperatur in einem Inertgasstrom wurde der nun pyrophore Katalysator unter Tridecanol (Hydrierprodukt) abgefüllt. Der so hergestellte Katalysator enthielt 21 Massen-% Nickel bezogen auf die Masse des reduzierten Katalysators.

### Beispiel 4 : Herstellung von Kobalt -Trägerkatalysatoren (gemäß der Erfindung)

Ein kommerzieller Aluminiumoxidträger (SP E538 der Fa. Axens) in Form von Extrudaten mit einem Durchmesser von etwa 1,6 mm und einer Länge von 4 bis 6 mm, der eine BET-Oberfläche von 270 m²/g (bestimmt nach dem BET-Verfahren durch Stickstoff-Adsorption gemäß DIN ISO 9277 wie im Beispiel 3 beschrieben) und ein Porenvolumen von 0,7 ml/g (bestimmt durch Quecksilber-Porosimetrie nach DIN 66133 wie im Beispiel 3 beschrieben) aufwies, wurde mit einer wässrigen Kobalt(II)nitrat-Lösung mit einem Kobaltgehalt von 22 Massen-% getränkt. Das während des Tränkens vom Träger aufgenommene Lösungsvolumen entsprach dabei etwa dem Porenvolumen des verwendeten Trägers. Anschließend wurde der mit Kobalt(II)nitrat-Lösung getränkte Träger bei 120 °C 12 h lang getrocknet und zunächst bei 300 °C in Luft kalziniert. Anschließend wurde der Katalysator bei 450 °C in einem Stickstoff/Wasserstoff-Gemisch, dessen Wasserstoffgehalt dabei von 5 bis auf 50 Volumen-% stufenweise erhöht wurde, aktiviert (reduziert). Nach Abkühlen auf Raumtemperatur wurde der nun pyrophore Katalysator unter Tridecanol (Hydrierprodukt) abgefüllt. Der so hergestellte Katalysator enthielt 18,5 Massen- % Kobalt bezogen auf die Masse des reduzierten Katalysators.

### Beispiel 5 (gemäß der Erfindung):

### C₁₃-Aldehydhydrierung bei 2,5 MPa / Kobalt-Katalysator gemäß Beispiel 4

Ein Liter eines Reaktionsaustrags der Rh-katalysierten Hydroformylierung von Tributen mit 15,89 Massen-% C₁₃-Aldehyd und 9,83 Massen-% Estern wurde in einer Kreislaufapparatur bei 180 °C und 2,5 MPa absolut an 100 g des Co-Katalysators auf γ-Al₂O₃-Träger mit 18,5 Massen -% Co gemäß Beispiel 4 in der Flüssigphase hydriert. Die Abgasmenge betrug 1 N1/h. Die Edukt- und Produkt-Analysen sind in Tabelle 3 wiedergegeben.

**Tabelle 3:**

| Versuchszeit (Stunden) | C₁₂-KWST (Massen-%) | C₁₃-al (Massen-%) | Formiat (Massen-%) | C₁₃-ol (Massen-%) | Hochsieder (Massen-%) |
|---|---|---|---|---|---|
| 0 | 8,10 | 15,89 | 9,83 | 66,21 | 0,18 |
| 0,5 | 8,05 | 2,90 | 3,01 | 85,79 | 0,25 |
| 1 | 8,18 | 1,34 | 0,51 | 89,62 | 0,35 |
| 3 | 8,17 | 0,93 | 0,30 | 89,92 | 0,68 |

Wie man aus der Tabelle 3 entnehmen kann, werden die Isotridecanylformiate bei der Hydrierung von Isotridecanal in Gegenwart von Kobalt-Katalysator sehr schnell und selektiv zum Wertprodukt Isotridecanol abgebaut. Im Vergleich zum Cu/Cr/Ni- Standardkatalysator (siehe Beispiele 1 und 2) werden in Gegenwart des erfindungsgemäßen Co-Katalysator deutlich höhere Ausbeuten erzielt.

### Beispiel 6 (gemäß der Erfindung):

### C₁₃-Aldehydhydrierung bei 2,5 MPa / Nickel-Katalysator gemäß Beispiel 3

Ein Liter eines Reaktionsaustrags der Rh-katalysierten Hydroformylierung von Tributen mit 14,54 Massen-% C₁₃-Aldehyd und 10,70 Massen-% Estern wurde in einer Kreislaufapparatur bei 180 °C und 2,5 MPa absolut an 100 g des Nickel-Katalysators auf γ-Al₂O₃-Träger gemäß Beispiel 3 mit 21 Massen -% Ni in der Flüssigphase hydriert. Die Abgasmenge betrug 1 Nl/h. Die Edukt- und Produkt-Analysen sind in Tabelle 4 wiedergegeben.

**Tabelle 4:**

| Versuchszeit (Stunden) | C₁₂-KWST (Massen-%) | C₁₃-al (Massen-%) | Formiat (Massen-%) | C₁₃-ol (Massen-%) | Hochsieder (Massen-%) |
|---|---|---|---|---|---|
| 0 | 8,28 | 14,54 | 10,70 | 66,29 | 0,19 |
| 0,5 | 8,43 * | 2,15 | 0,68 | 88,49 | 0,25 |
| 1 | 8,55 * | 1,18 | 0,53 | 89,39 | 0,35 |
| 3 | 9,93 * | 1,11 | 0,23 | 88,28 | 0,45 |

| | | | | | |
|---|---|---|---|---|---|
| * einschließlich C₁₃-KWST | | | | | |

Wie man aus der Tabelle 4 entnehmen kann, werden die Isotridecanylformiate bei der Hydrierung von Isotridecanal auch in Gegenwart von Nickel-Katalysator sehr schnell zum Wertprodukt Isotridecanol abgebaut. Im Unterschied zum Kobalt-Katalysator muss die Hydrierung am Nickel-Katalysator rechtzeitig beendet werden, um die Ausbeuteminderung durch Weiterreaktion zu vermeiden.

## Patentansprüche

1. Verfahren zur katalytischen Hydrierung von esterhaltigen Aldehydgemischen zu den entsprechenden Alkoholen,
**dadurch gekennzeichnet,**
**dass** ein Trägerkatalysator, der als Trägermaterial γ-Aluminiumoxid mit einer BET-Oberfläche von 70 bis 350 m²/g und als hydrieraktive Komponente 15 bis 25 Massen-% Nickel und/oder 5 bis 30 Massen-% Kobalt bezogen auf die Gesamtmasse des reduzierten Katalysators aufweist, wobei der Katalysator frei von Cu, Ru, Rh, Pd, Ag, Au, Pt, Ir und/oder Os ist und dass als esterhaltiges Aldehydgemisch ein Reaktionsgemisch aus der Hydroformylierung von Olefinen mit 6 bis 20 C-Atomen, welches einen Estergehalt von 3 bis 15 Massen-% aufweist, eingesetzt wird.

2. Verfahren nach Anspruch 1,
**dadurch gekennzeichnet,**
**dass** ein Trägerkatalysator eingesetzt wird, bei dem das Trägermaterial eine BET-Oberfläche von 110 bis 250 m²/g aufweist.

3. Verfahren nach Anspruch 1 oder 2,
**dadurch gekennzeichnet,**
**dass** der Katalysator 15 bis 25 Massen-% Kobalt enthält.

4. Verfahren nach einem der Ansprüche 1 bis 3,
**dadurch gekennzeichnet,**
**dass** die Hydrierung bei einem Druck von 0,5 bis 25 MPa durchgeführt wird.

5. Verfahren nach Anspruch 4,
**dadurch gekennzeichnet,**
**dass** die Hydrierung bei einem Druck von 2 bis 3 MPa durchgeführt wird.

6. Verfahren nach einem der Ansprüche 1 bis 5,
**dadurch gekennzeichnet,**
**dass** die Hydrierung bei einer Temperatur von 120 bis 220 °C durchgeführt wird.

7. Verfahren nach Anspruch 6,
**dadurch gekennzeichnet,**
**dass** die Hydriertemperatur 160 bis 190 °C beträgt.

8. Verfahren nach zumindest einem der Ansprüche 1 bis 7,
**dadurch gekennzeichnet,**
**dass** die Hydrierung unter Zusatz von Wasser zum esterhaltigen Aldehydgemisch durchgeführt wird.

9. Verfahren nach zumindest einem der Ansprüche 1 bis 7,
**dadurch gekennzeichnet,**
**dass** die Hydrierung ohne Zusatz von Wasser zum esterhaltigen Aldehydgemisch durchgeführt wird.

10. Verfahren nach einem der Ansprüche 1 bis 9,
**dadurch gekennzeichnet,**
**dass** als Aldehydgemische Reaktionsgemische aus der Hydroformylierung von Olefinen mit 8 bis 18 C-Atomen hydriert werden.

11. Verfahren nach einem der Ansprüche 1 bis 10,
**dadurch gekennzeichnet,**
**dass** als Aldehydgemische Reaktionsgemische aus der Hydroformylierung von Di-n-buten, Tri-n-buten und Tetra-n-buten hydriert werden.

## Claims

1. Process for the catalytic hydrogenation of ester-containing aldehyde mixtures to give the corresponding alcohols, **characterized in that** a supported catalyst which has γ-alumina having a BET surface area of from 70 to 350 m²/g as support material and from 15 to 25% by mass of nickel and/or from 5 to 30% by mass of cobalt, based on the total mass of the reduced catalyst, as a component having hydrogenation activity, where a catalyst is free from Cu, Ru, Rh, Pd, Ag, Au, Pt, Ir and/or Os, and **in that** a reaction mixture from the hydroformylation of olefins having 6 to 20 C atoms, which has an ester content of from 3 to 15% by mass, is used as the ester-containing aldehyde mixture.

2. Process according to Claim 1, **characterized in that** a supported catalyst in which the support material has a BET surface area of from 110 to 250 m²/g is used.

3. Process according to Claim 1 or 2, **characterized in that** the catalyst contains from 15 to 25% by mass of cobalt.

4. Process according to any of Claims 1 to 3, **characterized in that** the hydrogenation is carried out at a pressure of from 0.5 to 25 MPa.

5. Process according to Claim 4, **characterized in that** the hydrogenation is carried out at a pressure of from 2 to 3 MPa.

6. Process according to any of Claims 1 to 5, **characterized in that** the hydrogenation is carried out at a temperature of from 120 to 220°C.

7. Process according to Claim 6, **characterized in that** the hydrogenation temperature is from 160 to 190°C.

8. Process according to at least one of Claims 1 to 7, **characterized in that** the hydrogenation is carried out with addition of water to the ester-containing aldehyde mixture.

9. Process according to at least one of Claims 1 to 7, **characterized in that** the hydrogenation is carried out without addition of water to the ester-containing aldehyde mixture.

10. Process according to any of Claims 1 to 9, **characterized in that** reaction mixtures from the hydroformylation of olefins having 8 to 18 C atoms are hydrogenated as aldehyde mixtures.

11. Process according to any of Claims 1 to 10, **characterized in that** reaction mixtures from the hydroformylation of di-n-butene, tri-n-butene and tetra-n-butene are hydrogenated as aldehyde mixtures.

## Revendications

1. Procédé pour l'hydrogénation catalytique de mélanges d'aldéhydes contenant des esters en alcools correspondants, **caractérisé en ce qu'**un catalyseur supporté, qui présente comme matériau support du γ-oxyde d'aluminium présentant une surface BET de 70 à 350 m²/g et comme composant actif en hydrogénation 15 à 25% en masse de nickel et/ou 5 à 30% en masse de cobalt, par rapport à la masse totale du catalyseur réduit, le catalyseur étant exempt de Cu, de Ru, de Rh, de Pd, d'Ag, d'Au, de Pt, d'Ir et/ou d'Os et **en ce qu'**on utilise comme mélange d'aldéhydes contenant des esters un mélange réactionnel provenant de l'hydroformylation d'oléfines comprenant 6 à 20 atomes de carbone, qui présente une teneur en esters de 3 à 15% en masse.

2. Procédé selon la revendication 1, **caractérisé en ce qu'**on utilise un catalyseur supporté dans lequel le matériau support présente une surface BET de 110 à 250 m²/g.

3. Procédé selon la revendication 1 ou 2, **caractérisé en ce que** le catalyseur contient 15 à 25% en masse de cobalt.

4. Procédé selon l'une quelconque des revendications 1 à 3, **caractérisé en ce que** l'hydrogénation est réalisée à une pression de 0,5 à 25 MPa.

5. Procédé selon la revendication 4, **caractérisé en ce que** l'hydrogénation est réalisée à une pression de 2 à 3 MPa.

6. Procédé selon l'une quelconque des revendications 1 à 5, **caractérisé en ce que** l'hydrogénation est réalisée à une température de 120 à 220°C.

7. Procédé selon la revendication 6, **caractérisé en ce que** la température d'hydrogénation est de 160 à 190°C.

8. Procédé selon au moins l'une quelconque des revendications 1 à 7, **caractérisé en ce que** l'hydrogénation est réalisée avec addition d'eau au mélange d'aldéhydes contenant des esters.

9. Procédé selon au moins l'une quelconque des revendications 1 à 7, **caractérisé en ce que** l'hydrogénation est réalisée sans addition d'eau au mélange d'aldéhydes contenant des esters.

10. Procédé selon l'une quelconque des revendications 1 à 9, **caractérisé en ce que** des mélanges réactionnels provenant de l'hydroformylation d'oléfines comprenant 8 à 18 atomes de carbone sont hydrogénés en tant que mélanges d'aldéhydes.

11. Procédé selon l'une quelconque des revendications 1 à 10, **caractérisé en ce que** des mélanges réactionnels provenant de l'hydroformylation de di-n-butène, de tri-n-butène et de tétra-n-butène sont hydrogénés en tant que mélanges d'aldéhydes.
